# FASCICULE DE BREVET EUROPEEN

(11) **EP 0 630 654 B1**
(45) Date de publication et mention de la délivrance du brevet: **15.07.1998**
(21) Numéro de dépôt: 94401016.4
(22) Date de dépôt: 09.05.1994
(51) Int. Cl.: A61M 1/02, B01F 11/00

(54) **Appareil de prélèvement automatique de sang incorporant un dispositif d'agitation et de pesée**
Apparat zur automatischen Blutprobeentnahme mit einer Vorrichtung zum Wiegen und Rühren
Automatic blood sample device with weighing and agitating means

(30) Priorité: 13.05.1993 FR 9305761
(43) Date de publication de la demande: 28.12.1994
(73) Titulaire: ASSOCIATION POUR L'ESSOR DE LA TRANSFUSION SANGUINE DANS LA REGION DU NORD, 59000 Lille (FR)
(72) Inventeur: de Baudus, Jacques, F-59800 Lille (FR); Sanchez, Enriqué, F-62220 Carvin (FR); Depoortere, Jacques, F-59493 Villeneuve d'Ascq (FR); Bouzin, Georges, F-59491 Villeneuve d'Ascq (FR)
(74) Mandataire: Lhuillier, René

(56) Documents cités:
- EP-A- 0 077 732
- EP-A- 0 295 850
- EP-A- 0 423 335
- US-A- 4 109 319

## Description

L'invention se rapporte à un appareil de prélèvement automatique de quantités standards de sang et concerne plus précisément un dispositif d'agitation et de pesée qui lui est associé.

On sait que le sang obtenu par prélèvement sur une personne doit être recueilli dans un contenant parfaitement stérile, -habituellement une poche en matière plastique-, et doit aussi être mélangé avec un anticoagulant stérile. Or cet anticoagulant ne peut être rajouté au sang après le prélèvement, ni au cours du prélèvement. Il doit donc se trouver déjà dans la poche, et au début du remplissage de cette dernière, il est bien évidemment en grand excès par rapport à la quantité en sang prélevé. Si le mélange du sang avec l'anticoagulant est trop rapide, il s'ensuit une fragilisation des principes actifs et en particulier des globules rouges et une moindre récupération de ceux-ci. A l'inverse, si l'agitation est trop lente, elle n'est pas efficace, alors il peut se former des micro-caillots de sang. Il est donc nécessaire d'assurer une agitation correcte de la poche de sang au cours du prélèvement de telle façon que le sang se mélange localement et progressivement à l'anticoagulant sans fragiliser les principes actifs et sans coaguler. Les agitateurs à basculement utilisés jusqu'à présent, tels que celui décrit dans le EP-A- 0 077 732, ne donnent pas toujours -de ce point de vue- les résultats escomptés.

Un autre impératif de ce genre d'appareil est d'effectuer un prélèvement automatique de la quantité de sang voulue et d'utiliser pour cela un système qui interrompt le remplissage quand le volume est atteint. A ce sujet on connait un dispositif de dosage qui met en oeuvre une cale d'épaisseur, c'est-à-dire une pièce qui réagit au gonflement de la poche en phase finale de remplissage pour manoeuvrer une vanne mécanique qui ferme le conduit de prélèvement. Mais du fait de l'élasticité variable des poches due à leurs formes non parfaitement homogènes, aux différences de qualités du plastique utilisé, aux faux-plis que peuvent en prendre les poches en cours de remplissage, les prélèvements obtenus sont imprécis et variables d'une poche à l'autre dans des proportions difficilement acceptables, car cela pose des problèmes de balourd lors des opérations ultérieures de centrifugation.

Pour éviter cet inconvénient il a été imaginé un dispositif de pesée par jauges de contrainte, lié à un mécanisme d'agitation à plat des poches de sang prélevé, du genre décrit dans le US-A- 4 109 319, l'ensemble s'intégrant dans un appareil qui résoud les divers problèmes évoqués précédemment et autorisant des prélèvements automatiques précis et fiables, facilement programmables et qui de surcroît est d'un coût de réalisation faible et d'un encombrement réduit.

Un objet principal de la présente invention consiste donc en un appareil de prélèvement automatique de sang qui est recueilli dans une poche renfermant un produit anticoagulant et reposant sur une nacelle associée à un mécanisme d'agitation, entraîné en un mouvement orbital par la rotation d'une pièce excentrique mue par un moteur, l'appareil étant équipé d'un dispositif de programmation à microprocesseur, appareil selon lequel le mécanisme d'agitation est constitué d'une plaque horizontale susceptible de glisser à plat sur un plateau fixe, lequel dispose d'un organe de guidage de la plaque, la pièce excentrique étant située hors de la surface définie par le plateau et coopérant avec un anneau relié à la plaque, et ledit plateau fixe étant directement associé à un capteur de force assurant à intervalles réguliers la pesée de la poche de sang en cours de remplissage.

En outre l'organe de guidage du plateau peut être un axe vertical qui traverse un trou oblong prévu au centre de la plaque.

Selon encore une autre caractéristique particulière de l'invention, la pièce excentrique est une came montée sur un arbre d'un moteur, ladite came étant engagée dans l'anneau relié à la plaque par une patte. Le capteur de force à jauges de contrainte est avantageusement placé entre un plateau inférieur servant de socle et le plateau fixe sur lequel glisse directement la plaque horizontale.

D'autres caractéristiques particulières et avantages de l'invention ressortiront de la description qui va suivre d'un exemple non limitatif de réalisation faisant référence aux dessins annexés qui représentent :
- figure 1 une vue schématique en perspective du mécanisme de l'appareil
- figures 2 et 3 des vues respectivement du dessus et de côté dudit mécanisme
- figure 4 une vue en perspective éclatée de l'ensemble de l'appareil.

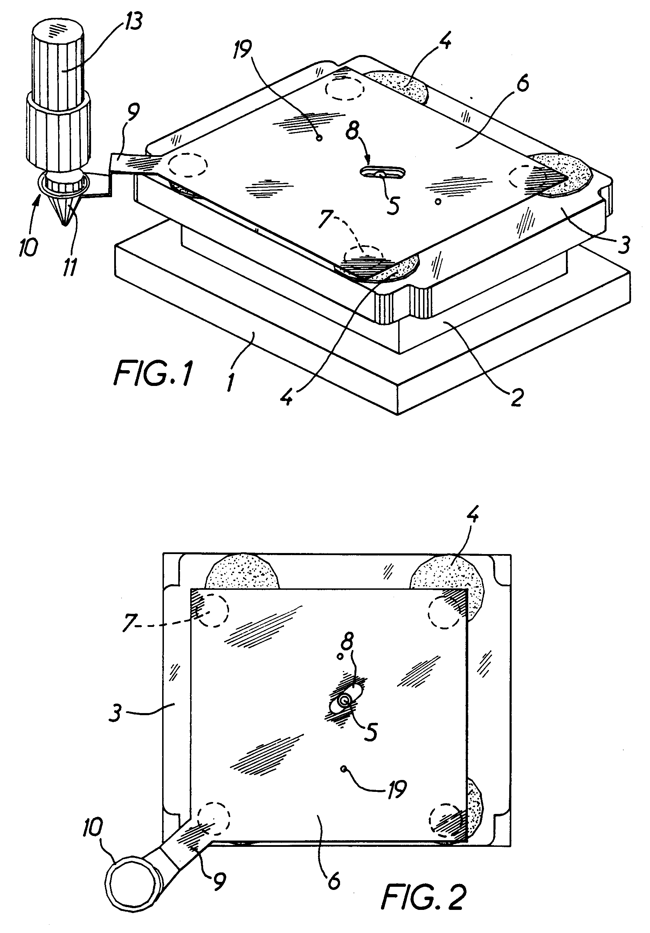

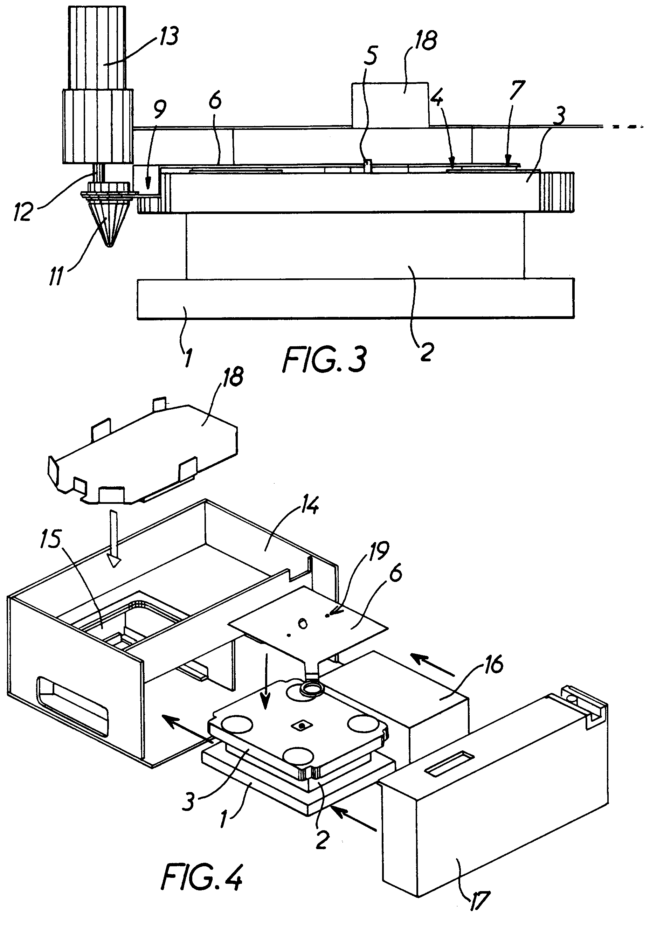

L'appareil représenté à la figure 1 se compose essentiellement d'un plateau inférieur 1 en partie basse servant de socle, sur lequel est monté un capteur de force 2 à jauges de contraintes, lui-même coiffé d'un autre plateau supérieur 3. Ces trois pièces sont des organes fixes logés à la base d'un boîtier 14 (visible à la figure 4) qui constitue le corps de l'appareil.

Sur le plateau supérieur 3 sont collés quatre patins 4 réalisés en un matériau ayant un faible coefficient de friction, et en son centre est fixé un petit axe vertical 5. Une plaque en acier inoxydable 6 est disposée au-dessus du plateau supérieur 3. Elle est équipée sur sa face inférieure de quatre patins 7 également en matériau à faible coefficient de friction, grâce auxquels elle est en appui sur les patins 4. La plaque peut ainsi aisément glisser à plat sur le plateau supérieur. En faisant référence également aux figures 2 et 3 on voit que la plaque est pourvue en son centre d'un trou oblong 8 que traverse l'axe 5. Elle est également munie de deux trous 19 dans lesquels s'engagent des ergots d'accouplements d'une nacelle 18 destinée à recevoir la poche de sang. Une extrémité de la plaque 6 est reliée par une patte 9 à un anneau 10 dans lequel s'engage une came 11. Cet anneau enserre une collerette en matériau à faible coefficient de friction. La came est montée de façon excentrique sur un arbre 12 entrainé en rotation par un moteur 13. On notera que ce moteur est fixe par rapport à l'appareil et monté sur un support approprié non représenté.

On comprend que la rotation de l'arbre 12 va -du fait de la rotation de la came 11 qui tourne dans l'anneau 10-, provoquer un mouvement orbital à plat de la plaque 16 qui glisse grâce aux patins sur le plateau supérieur 3. Ce mouvement est guidé par le coulissement de l'axe 5 dans le trou oblong 8.

L'appareil visible à la figure 4 a la forme d'un boîtier parallèlipipédique 14 à l'intérieur duquel vient se loger dans un réceptacle 15 l'ensemble des plateaux 1,2 et 3. La plaque 6 affleure au sommet du réceptacle. Le boîtier 14 renferme aussi une batterie 16 qui le rend autonome et un dispositif à microprocesseur 17 pour la commande et la programmation du fonctionnement, avantageusement rapporté à l'avant. On voit que ces différents éléments se juxtaposent à l'intérieur du boîtier 14 de l'appareil selon les flèches. Ils restent en outre facilement accessibles et amovibles, ce qui facilite leur contrôle et leur entretien. L'appareil de faible dimension s'installe aisément à proximité d'un poste de prélèvement. La poche de prélèvement est reliée par une tubulure souple à l'aiguille de ponction et une vanne ou pince péristaltique permet de pincer la tubulure et par conséquent d'arrêter le prélèvement.

L'appareil fonctionne de la façon suivante. On dispose d'abord sur la nacelle 18 une poche de prélèvement renfermant sa dose d'anticoagulant, raccordée à la tubulure de prélèvement terminée par l'aiguille, ladite tubulure étant placée dans la pince péristaltique qui est en position ouverte. Ensuite l'opérateur commande la fermeture de la vanne péristaltique puis ponctionne la veine du donneur. Avant de commencer le prélèvement, l'opérateur appuie sur une touche du dispositif de commande pour provoquer l'opération de prise en compte et de mémorisation de la tare c'est-à-dire le poids de la poche vide, le poids de l'anticoagulant qu'elle contient et le poids de la tubulure portée par la plaque. Puis on affiche le poids de la quantité de sang à prélever. Le logiciel commande l'ouverture de la vanne péristaltique et le prélèvement commence. Simultanément le moteur 13 provoque l'agitation orbitale du plateau pendant une durée déterminée, par exemple cinq secondes. Puis le cycle d'agitation s'interrompt par exemple durant trois secondes. Durant cette phase d'arrêt est assurée, grâce au capteur de force 2, la mesure de la masse de sang prélevé. Quant on arrive à une mesure approchante prédéterminée de la valeur de consigne -par exemple à trente grammes- l'agitation s'arrête et le microprocesseur mesure alors la masse en permanence. Dès que la valeur de consigne de la masse prélevée est atteinte la vanne péristaltique se met en position fermée et un signal sonore retentit. Le prélèvement est donc terminé.

Grâce au microprocesseur on peut sélectionner la masse de sang à prélever à choisir par l'opérateur, on peut prévenir l'opérateur lorsque le débit de sang est insuffisant (par exemple moins de 2,5 ml par dix secondes) et on peut aussi faire la conversion de la masse prélevée en volume prélevé.

Il est prévu aussi une mise en sécurité de l'appareil en cas de défaillance de la batterie d'alimentation.

## Revendications

1. Appareil de prélèvement automatique de sang qui est recueilli dans une poche renfermant un produit anticoagulant et reposant sur une nacelle associée à un mécanisme d'agitation entraîné en un mouvement orbital par la rotation d'une pièce excentrique (11) mue par un moteur (13), ledit mécanisme d'agitation étant constitué d'une plaque horizontale (6) susceptible de glisser à plat sur un plateau fixe (3), lequel dispose d'un organe de guidage (5) de la plaque, caractérisé en ce que la pièce excentrique (11) est située hors de la surface définie par le plateau (3) et coopère avec un anneau (10) relié à la plaque (6) et en ce que ledit plateau fixe (3) est directement associé à un capteur de force (2) assurant à intervalles réguliers la pesée de la poche de sang en cours de remplissage et en ce que l'appareil est équipé d'un dispositif de programmation à microprocesseur.

2. Appareil de prélèvement selon la revendication 1, caractérisé en ce que l'organe de guidage du plateau (3) est un axe vertical (5) qui traverse un trou oblong (8) prévu au centre de la plaque (6).

3. Appareil de prélèvement selon la revendication 1, caractérisé en ce que la pièce excentrique est une came (11) montée sur un arbre (12) du moteur (13), ladite came étant engagée dans l'anneau (10) relié à la plaque (6) par une patte (9).

4. Appareil de prélèvement selon la revendication 1, caractérisé en ce que le capteur de force (2) à jauges de contrainte est placé entre un plateau inférieur (1) servant de socle et le plateau fixe (3) sur lequel glisse directement la plaque horizontale (6).

5. Appareil de prélèvement selon la revendication 1, caractérisé en ce que l'ensemble fixe formé du plateau inférieur (1), du capteur de force (2), du plateau fixe (3) avec sa plaque mobile (6) ainsi qu'une batterie (16) et un dispositif à microprocesseur (17) sont juxtaposés à l'intérieur d'un même boîtier (14) formant le corps de l'appareil.

## Claims

1. Device for automatically sampling blood which is collected in a bag containing an anticoagulant product and resting on a boat member associated with an agitating mechanism driven in orbital movement through the rotation of an eccentric part (11) driven by a motor (13), the said agitating mechanism being constituted by a horizontal plate (6) capable of sliding flat on a fixed bedplate (3), which is provided with a member (5) for guiding the plate, characterised in that the eccentric part (11) is located outside the surface defined by the bedplate (3) and co-operates with a ring (10) connected to the plate (6), in that the said fixed bedplate (3) is directly associated with a force meter (2) ensuring the weighing, at regular intervals, of the blood bag in the process of being filled, and in that the device is equipped with a microprocessor type programming device.

2. Sampling device according to claim 1, characterised in that the member for guiding the bedplate (3) is a vertical pin (5) which passes through an oblong hole (8) provided in the centre of the plate (6).

3. Sampling device according to claim 1, characterised in that the eccentric part is a cam (11) mounted on a shaft (12) of the motor (13), the said cam being engaged in a ring (10) connected to the plate (6) by an arm (9).

4. Sampling device according to claim 1, characterised in that the strain gauge type force meter (2) is placed between a lower bedplate (1), serving as a base, and the fixed bedplate (3), over which the horizontal plate (6) slides directly.

5. Sampling device according to claim 1, characterised in that the fixed assembly formed by the lower bedplate (1), the force meter (2), the fixed bedplate (3), with its moving plate (6), as well as a battery (16) and a microprocessor device (17) are juxtaposed inside one and the same housing (14) forming the body of the device.

## Patentansprüche

1. Apparat zur automatischen Entnahme von Blut, das in einem ein antikoagulierendes Produkt umschließenden Beutel aufgefangen wird, der auf einem einem Rührmechanismus zugeordneten Schiffchen ruht, wobei der Rührmechanismus durch die Drehung eines mit einem Motor (13) versehenen exzentrischen Teils (11) zur Ausführung einer Orbitalbewegung angetrieben wird und durch eine horizontale Scheibe (6) gebildet wird, die liegend auf einer feststehenden Platte (3) gleiten kann, welche ein Glied (5) zur Führung der Scheibe aufweist, dadurch gekennzeichnet, daß sich das exzentrische Teil (11) außerhalb der durch die Platte (3) definierten Fläche befindet und mit einem mit der Scheibe (6) verbundenen Ring (10) zusammenwirkt und daß die feststehende Platte (3) direkt einem Meßwertgeber (2) zugeordnet ist, der während des Füllens in regelmäßigen Abständen das Wiegen des Blutbeutels gewährleistet, und daß der Apparat mit einer Mikroprozessor-Programmiervorrichtung ausgestaltet ist.

2. Entnahmeapparat nach Anspruch 1, dadurch gekennzeichnet, daß es sich bei dem Glied zur Führung der Platte (3) um eine vertikale Achse (5) handelt, die ein in der Mitte der Scheibe (6) vorgesehenes Langloch (8) durchquert.

3. Entnahmeapparat nach Anspruch 1, dadurch gekennzeichnet, daß es sich bei dem exzentrischen Teil um einen an einer Welle (12) des Motors (13) angebrachten Nocken (11) handelt, der in den Ring (10) eingreift, welcher durch einen Ansatz (9) mit der Scheibe (6) verbunden ist.

4. Entnahmeapparat nach Anspruch 1, dadurch gekennzeichnet, daß der Dehnungsmeßwertgeber (2) zwischen einer unteren Platte (1), die als Sockel dient, und der feststehenden Platte (3), auf der die horizontale Scheibe (6) direkt gleitet, angeordnet ist.

5. Entnahmeapparat nach Anspruch 1, dadurch gekennzeichnet, daß der durch die untere Platte (1), den Meßwertgeber (2), die feststehende Platte (3) mit ihrer beweglichen Scheibe (6) gebildete feststehende Aufbau sowie eine Batterie (16) und eine Mikroprozessorvorrichtung (17) nebeneinander im Innern des gleichen den Körper des Apparats bildenden Gehäuses (14) angeordnet sind.
